# EUROPÄISCHE PATENTANMELDUNG

(11) **EP 0 694 534 A1**
(43) Veröffentlichungstag der Anmeldung: **31.01.1996**
(21) Anmeldenummer: 95110973.5
(22) Anmeldetag: 13.07.1995
(51) Int. Cl.: C07D 215/48, C07D 401/04, A61K 31/47

(54) **3-Substituierte Chinolin-5-carbonsäurederivate und Verfahren zu ihrer Herstellung**

(30) Priorität: 26.07.1994 DE 4426373
(71) Anmelder: BAYER AG, D-51368 Leverkusen (DE)
(72) Erfinder: Stoltefuss, Jürgen, D-42781 Haan (DE); Negele, Michael, Dr., D-42697 Solingen (DE); Wahl, Karl-Heinz, Dr., D-51519 Odenthal (DE); Lenfers, Jan-Bernd, Dr., D-42117 Wuppertal (DE); Samaan, Samir, Prof.Dr., D-42113 Wuppertal (DE)

(57) **Zusammenfassung**

Die vorliegende Erfindung betrifft 3-substituierte Chinolin-5-carbonsäurederivate und Verfahren zu ihrer Herstellung.

## Beschreibung

Die vorliegende Erfindung betrifft 3-substituierte Chinolin-5-carbonsäurederivate und Verfahren zu ihrer Herstellung.

Aus der Publikation J. Med. Chem. 16, 118 (1973) ist die Verbindung 3-Phenylchinolin-5-carbonsäureethylester bekannt, die durch Kondensation von 3-Aminobenzoesäureethylester mit 1,3-Diethoxy-2-phenylpropan-2-ol erhalten wird.

Aus den Publikationen von G. Pagani et al.; Farmaco, Ed. Sci. 29 (7), 507-16 (1974); T.S. Tulyaganow et al.; Khim. Prir. Soedin. (5), 635-8 (1982); S.D. Sharma et al.; Indian J. Chem. Sect. B, 27B (5), 494-7 (1988) sind einige Chinolin-N- und N,N-alkylamide bekannt. Auch Chinolin-5-carbonnitril wurde bereits beschrieben (vgl. hierzu EP 452 712; Pharmazie, 31 (3), 145- (1976)).

Die vorliegende Erfindung betrifft 3-substituierte Chinolin-5-carbonsäurederivate der allgemeinen Formel (I) 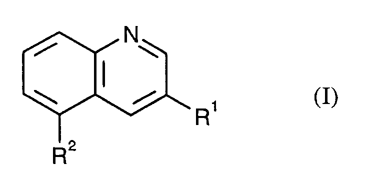
in welcher
- R¹: für geradkettiges oder verzweigtes Alkyl mit bis zu 12 Kohlenstoffatomen steht, oder für einen Rest der Formel -X-R³ steht,
worin
- X: eine einfache Bindung, ein Sauerstoff- oder Schwefelatom oder eine Alkylidenkette mit bis zu 6 C-Atomen bedeutet,
und
- R³: Cycloalkyl mit 3 bis 8 Kohlenstoffatomen oder Phenyl bedeutet, das gegebenenfalls bis zu 3-fach gleich oder verschieden durch Nitro, Halogen, Trifluormethyl oder durch geradkettiges oder verzweigtes Alkyl, Alkoxy oder Alkoxycarbonyl mit jeweils bis zu 8 Kohlenstoffatomen, Hydroxy oder Carboxy substituiert ist, und
- R²: für einen Rest aus der Gruppe -COOCH₃, -CN oder -CONH₂ steht.

Bevorzugt sind Verbindungen der allgemeinen Formel (I),
in welcher
- R¹: für geradkettiges oder verzweigtes Alkyl mit bis zu 10 Kohlenstoffatomen steht, oder
für einen Rest der Formel X-R³ steht,
worin
- X: eine einfache Bindung, ein Sauerstoff- oder Schwefelatom oder eine Alkylidenkette mit bis zu 4 C-Atomen bedeutet
und
- R³: Cyclopropyl, Cyclopentyl, Cyclohexyl oder Phenyl bedeutet, das gegebenenfalls bis zu 2-fach gleich oder verschieden durch Nitro, Fluor, Chlor, Brom, Trifluormethyl oder durch geradkettiges oder verzweigtes Alkyl, Alkoxy oder Alkoxycarbonyl mit jeweils bis zu 6 Kohlenstoffatomen substituiert ist, und
- R²: für einen Rest aus der Gruppe -COOCH₃, -CN oder -CONH₂ steht.

Besonders bevorzugt sind Verbindungen der allgemeinen Formel (I),
in welcher
- R¹: für geradkettiges oder verzweigtes Alkyl mit bis zu 6 Kohlenstoffatomen steht oder für Cyclohexyl, Cyclohexylmethyl, Cyclopentyl steht oder für Phenyl steht, das gegebenenfalls bis zu 2-fach gleich oder verschieden durch Nitro, Fluor, Chlor, Trifluormethyl oder durch geradkettiges oder verzweigtes Alkyl, Alkoxy oder Alkoxycarbonyl mit jeweils bis zu 4 Kohlenstoffatomen substituiert ist, und
- R²: für einen Rest aus der Gruppe -COOCH₃, -CN oder -CONH₂ steht.

Die erfindungsgemäßen Verbindungen der allgemeinen Formel (I) werden hergestellt, indem man
[A] im Falle R² = COOCH₃ 3-substituierte Chinolin-5-carbonsäuren der allgemeinen Formel (II) 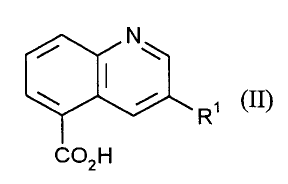 in welcher
   R¹ die oben angegebene Bedeutung hat,
   durch Umsetzung mit anorganischen oder organischen Säurechloriden wie Thionylchlorid oder Sulfurylchlorid, gegebenenfalls nach Isolierung der entsprechenden Carbonsäurechloride, diese mit Methanol verestert,
   oder
[B] Carbonsäureamide der allgemeinen Formel (III) 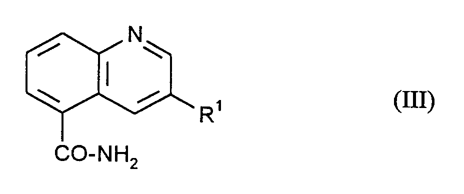 in welcher
   R¹ die oben angegebene Bedeutung hat,
   zunächst mit weitgehend wasserfreien Säuren in Methanol umsetzt und anschließend durch Zugabe von Basen die Ester ausfällt,
   oder
[C] im Falle R² = -CO-NH₂ 4-Amino-3-hydroxyphthalimidin der Formel (IV) 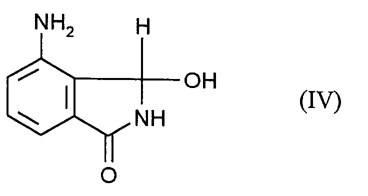 mit Aldehyden oder deren Acetal-Derivaten der allgemeinen Formel (Va) bzw. (Vb)

   R¹-CH₂-CHO (Va)

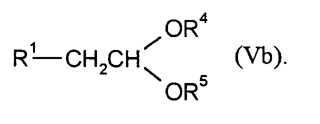

   in welchen
   - R¹: die oben angegebene Bedeutung hat,
   - R⁴ und R⁵: gleich oder verschieden sind und für Cycloalkyl mit 3 bis 6 Kohlenstoffatomen, Aryl mit 6 bis 10 Kohlenstoffatomen, oder für geradkettiges oder verzweigtes Alkyl mit bis zu 6 Kohlenstoffatomen stehen, oder gemeinsam für eine Ethylen- oder Propylidengruppe stehen,
   in inerten Lösemitteln umsetzt und
[D] im Falle R² = -CN
   die Amide der Formel III gegebenenfalls in Anwesenheit von inerten Lösemitteln durch Behandlung mit wasserentziehenden Mitteln in die Nitrile überführt.

Die erfindungsgemäßen Verfahren können durch folgende Formelschemata beispielhaft erläutert werden: 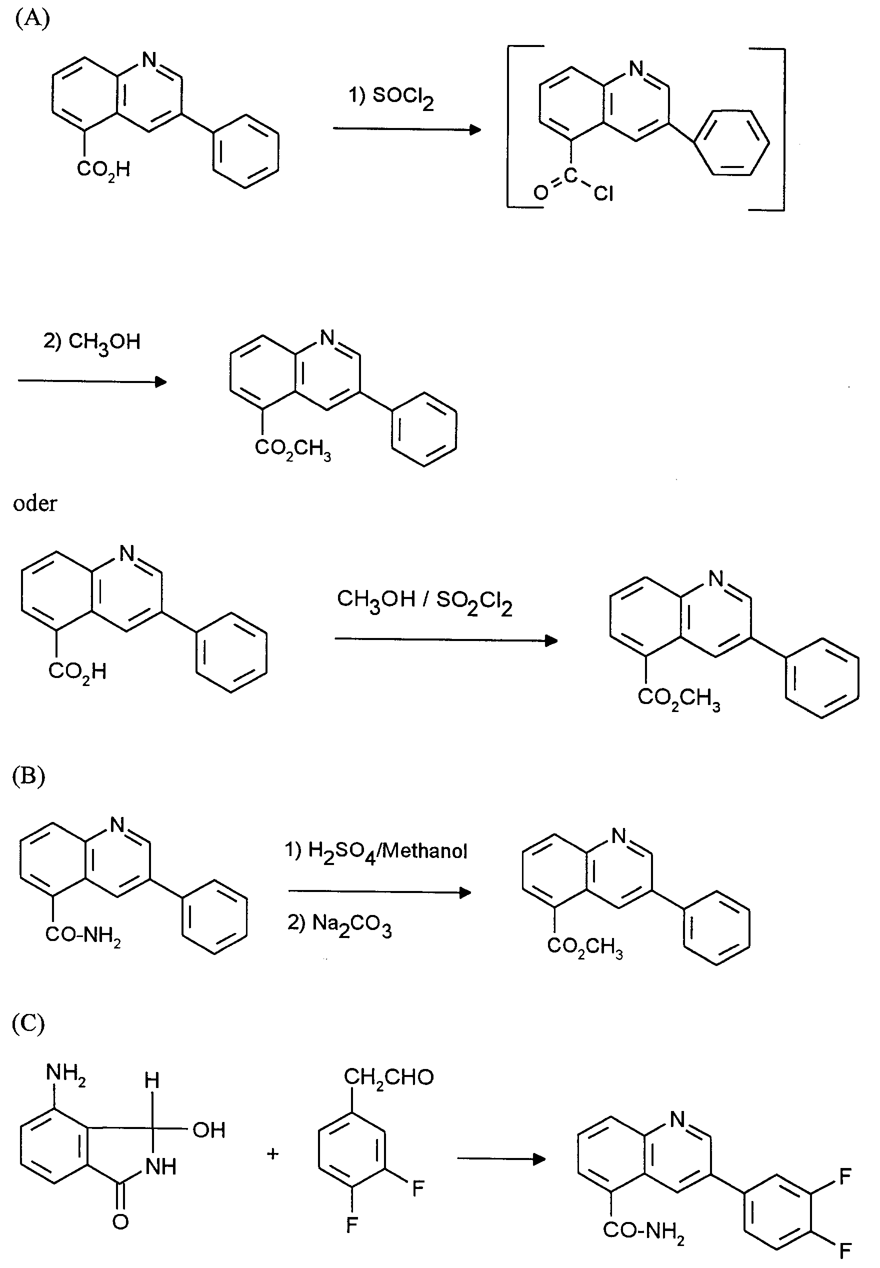 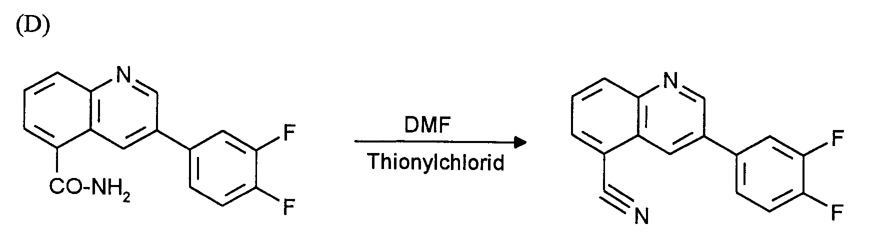
Als Lösemittel für das Verfahren [A] eignen sich hierbei für den ersten Schritt alle inerten organischen Lösemittel, die sich unter den Reaktionsbedingungen nicht verändern. Hierzu gehören Acetonitril, oder Amide wie Hexamethylphosphorsäuretriamid oder Dimethylformamid, oder halogenierte Kohlenwasserstoffe wie Methylenchlorid, Tetrachlorkohlenstoff oder Kohlenwasserstoffe wie Benzol, Chlorbenzol oder Toluol. Ebenso ist es möglich, Gemische der genannten Lösemittel zu verwenden. Bevorzugt sind Dimethylformamid und Toluol.

Als Säurechloride eignen sich im allgemeinen die üblichen anorganischen oder organischen Säurechloride wie Thionylchlorid, Sulfurylchlorid, Phosphortrichlorid, Phosphorpentachlorid, Oxalylchlorid oder Phthalsäuredichlorid. Bevorzugt sind Thionylchlorid, Sulfurylchlorid und Oxalylchlorid. Gegebenenfalls kann auch das Säurechlorid als Lösemittel fungieren.

Die Reaktionstemperaturen für den ersten Schritt können in einem größeren Bereich variiert werden. Im allgemeinen arbeitet man zwischen +20°C und +250°C, vorzugsweise zwischen 60°C und 160°C, insbesondere bei der Siedetemperatur des betreffenden Lösemittels.

Die Umsetzungen können bei Normaldruck, aber auch bei erhöhtem Druck (z.B. 1 bis 100 bar) durchgeführt werden.

In einer Variante ist es möglich, die Carbonsäuren der allgemeinen Formel (II) in siedendem Methanol mit einer überstöchiometrischen Menge Sulfurylchlorid zu versehen und den gebildeten Methylester in einem zweiten Schritt durch Einrühren des Reaktionsgemisches in wäßrigen Basen auszufällen.

Als Basen eignen sich im allgemeinen Alkali- und Erdalkalihydroxide wie beispielsweise Natrium- und Kaliumhydroxid, oder Alkali- und Erdalkalicarbonate wie beispielsweise Natrium- und Kaliumcarbonat. Bevorzugt sind Natriumhydroxid und Natriumcarbonat.

Die Base wird im allgemeinen in einer Menge von 2 mol bis 8 mol, bevorzugt von 2 mol bis 5 mol, jeweils bezogen auf 1 mol der Verbindungen der allgemeinen Formel (II) eingesetzt.

Das Verfahren [B] wird bevorzugt in Methanol durchgeführt.

Als weitgehend wasserfreie anorganische Säuren eignen sich gasförmige Salzsäure, gasförmiger Bromwasserstoff, konzentrierte Schwefelsäure oder konzentrierte Phosphorsäure. Bevorzugt ist die Umsetzung konzentrierter Schwefelsäure bzw. HCl-Gas.

Die anorganische Säure wird in einer Menge von 2 mol bis 8 mol, bevorzugt von 2 mol bis 5 mol, bezogen auf 1 mol der Verbindung der allgemeinen Formel (IV) eingesetzt.

Als Basen für die Ausfällung des entsprechenden Methylesters eignen sich die oben aufgeführten Basen in Art und Menge, bezogen auf 1 mol des Amides der Formel (III) angepaßt auf die eingesetzte Säuremenge.

Die Reaktionstemperaturen können in einem größeren Bereich variiert werden. Im allgemeinen arbeitet man zwischen +20°C und +250°C, vorzugsweise zwischen 40°C und 160°C, insbesondere bei der Siedetemperatur von Methanol bei Normaldruck.

Die Umsetzungen können bei Normaldruck, aber auch bei erhöhtem Druck (z.B. 1 bis 100 bar) durchgeführt werden.

Die Carbonsäuren der allgemeinen Formel (II) sind bekannt oder können nach üblichen Methoden hergestellt werden, indem man
4-Amino-3-hydroxyphthalid der Formel (V) 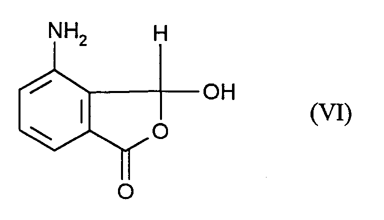
mit Aldehyden der allgemeinen Formel (Va) oder deren Acetale der allgemeinen Formel (Vb)

R¹-CH₂-CHO (Va)

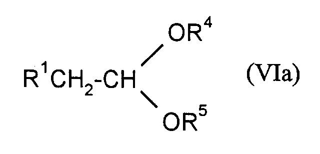

in welcher
R¹, R⁴ und R⁵ die oben angegebene Bedeutung haben,
in inerten organischen Lösemitteln umsetzt.

Als Lösemittel eignen sich alle unter den Reaktionsbedingungen inerten Lösemittel. Bevorzugt sind Methanol, Isopropanol, Ethanol und n-Propanol, Acetonitril, Tetrahydrofuran, Diethylenglykoldimethylether und Essigsäure. Besonders bevorzugt sind Ethanol und Isopropanol.

Die Reaktionstemperaturen können in einem größeren Bereich variert werden. Im allgemeinen arbeitet man zwischen +20°C und +150°C, vorzugsweise zwischen +60°C und +120°C, insbesondere bei der Siedetemperatur des betreffenden Lösemittels.

Die Umsetzung kann bei Normaldruck, aber auch bei erhöhtem Druck (z.B 1 bis 50 bar) durchgeführt werden. Im allgemeinen arbeitet man bei Normaldruck.

Die Verbindung der allgemeinen Formel (VI) ist bekannt [vgl. EP 476 474 A1].

Die Aldehyde und deren Acetale der allgemeinen Formeln (Va) und (Vb) sind teilweise bekannt oder können nach bekannten Methoden hergestellt werden.

Als Lösemittel für das Verfahren (C) eignen sich hierbei alle inerten organischen Lösemittel, die sich unter den Reaktionsbedingungen nicht verändern und deren Gemische mit Wasser. Hierzu gehören Alkohole wie Methanol, Ethanol, Propanol, Isopropanol oder Ethylenglykole, oder Ether wie Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol, Chlorbenzol oder Toluol, sowie Ester wie beispielsweise Essigsäureethylester. Ebenso ist es möglich, Gemische der genannten Lösemittel zu verwenden. Bevorzugt sind Dimethylformamid, Essigsäure, Essigsäure/Wasser, Chlorbenzol oder Diethylenglykoldimethylether. Ferner kann in dem entsprechenden Aldehyd oder Acetal als Lösemittel gearbeitet werden. Besonders bevorzugt sind Essigsäure bzw. Gemische von Essigsäure mit Wasser.

Die Reaktionstemperaturen für den ersten Schritt können in einem größeren Bereich variiert werden. Im allgemeinen arbeitet man zwischen +20°C und +250°C, vorzugsweise zwischen 60°C und 160°C, insbesondere bei der Siedetemperatur des betreffenden Lösemittels.

Die Umsetzungen können bei Normaldruck, aber auch bei erhöhtem Druck (z.B. 1 bis 100 bar) durchgeführt werden.

Das 4-Amino-3-hydroxyphthalimidin der Formel (IV) ist neu und kann beispielsweise hergestellt werden, indem man das bekannte 4-Nitro-3-hydroxyphthalimidin in inerten Lösemitteln, bevorzugt durch Hydrierung, in Anwesenheit eines Katalysators, nach üblichen Methoden reduziert.

Als Lösungsmittel für das Verfahren (D) (R² = CN) eignen sich insbesondere Dimethylformamid oder Dimethylsulfoxid. Es ist aber auch möglich, das entsprechende wasserentziehende Mittel als Lösungsmittel einzusetzen.

Als wasserentziehende Mittel eignen sich beispielsweise Phosphorpentoxid, Phosphorylchlorid, Phosphorpentachlorid oder Thionylchlorid. Bevorzugt ist Thionylchlorid.

Die Reaktionstemperaturen für den zweiten Schritt liegen im allgemeinen in einem Bereich von -20°C bis +50°C, vorzugsweise bei 0°C - +25°C.

Die Umsetzungen können bei Normaldruck, aber auch bei erhöhtem Druck (z.B. 1 bis 100 bar) durchgeführt werden.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen 3-substituierten Chinolin-Verbindungen sind für die 1,4-Dihydropyridin-Chemie von großer Bedeutung, da sie wertvolle Zwischenprodukte zur Synthese von 4-Chinolyl-dihydropyridinen sind [vgl. hierzu z.B. EP 452 712].

### Herstellungsbeispiele

### Beispiel 1

### 3-Phenylchinolin-5-carbonsäuremethylester

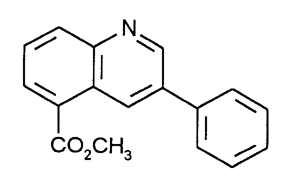

### 1a. Verfahren A: (1. Variante)

In eine Suspension von 187,5 g 3-Phenylchinolin-5-carbonsäure (0,75 mol) in 1500 ml Toluol tropft man während 30 Minuten bei 70°C - 75°C eine Lösung von 67,5 ml Thionylchlorid (0,93 mol) in 300 ml Toluol. Der Ansatz wird danach 2 Stunden unter Rückfluß gerührt, wobei die Temperatur auf ca. 110°C ansteigt. Nach Abkühlen auf 60°C werden 150 ml Methanol innerhalb von 10 Minuten zugetropft (leicht exotherm). Der Ansatz wird noch 30 Minuten unter Rückfluß (ca. 75°C) gerührt und danach auf Raumtemperatur abgekühlt. Zum Ansatz läßt man eine Lösung aus 150 g Natriumcarbonat in 900 ml Wasser laufen. Der pH-Wert muß jetzt 9,0 bis 9,5 betragen. Man läßt 30 Minuten bei ca. 40°C nachrühren und trennt die toluolische Phase ab. Diese Lösung wird über Na₂SO₄ getrocknet und nach Filtration entsprechend dem Anwendungsbeispiel (Variante a) auf tert. Butanol durch Abziehen des Toluols umgestellt.

### 1b. Verfahren A: (2. Variante)

In 2 ltr. Methanol, (techn.) werden 249,3 g (1 mol) 3-Phenylchinolin-5-carbonsäure unter Rühren bei ca. 50°C suspendiert. Innerhalb von ca. 2 h werden bei ca. 50-60°C (exotherme Reaktion, Heizbad entfernen) 136 ml Sulfurylchlorid (1,7 mol) zugetropft. Nach vollendeter Zugabe wird über Nacht am Rückfluß gekocht und anschließend auf Raumtemperatur abgekühlt. Das Reaktionsgemisch wird in ca. 15 min in eine Lösung von 188 g (1,77 mol) Na₂CO₃ in 2 ltr. Wasser getropft; der Niederschlag abgesaugt, mit 500 ml Wasser salzfrei gewaschen und über Nacht im Vakuum bei 60°C getrocknet.
Ausbeute: 253 g (96% d.Th.)
Fp.: 99-100°C

### 1c. Verfahren B:

In 2,0 ltr. Methanol (techn.) werden 248,0 g (1 mol) 3-Phenylchinolin-5-carbonamid unter Rühren am Rückfluß suspendiert. Innerhalb von ca. 2 h werden 300 ml konz. H₂SO₄ zugetropft. Anschließend wird noch ca. 4-5 h am Rückfluß nachgerührt; es entsteht eine klare Lösung. Zur Aufarbeitung wird die auf Raumtemperatur abgekühlte Lösung in 4 ltr. eiskalter wäßriger Soda-Lösung (ca. 4 molar) eingerührt. Der Methylester fällt aus, wird 1 h nachgerührt und abgesaugt. Der Filterkuchen wird mit 500ml H₂O neutral gewaschen und anschließend im Vakuum bei ca. 80°C über Nacht getrocknet.
Ausbeute (roh): 262 g (99,5% d.Th.)
Das Rohprodukt wird aus 2 ltr. Methanol umkristallisiert.
Ausbeute (nach Trocknen): 204 g (77,5% d.Th.)
Fp.: 99-100°C

### Beispiel 2

### 3-Phenylchinolin-5-carbonsäureamid

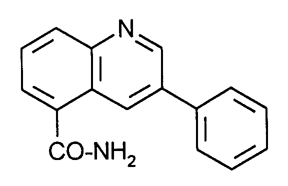

### Verfahren (C)

a)
   3,28 g (20 mmol) 4-Amino-3-hydroxyphthalimidin werden in 40 ml Diglyme suspendiert und mit 4 ml Phenylacetaldehyd versetzt. Es wird 1 Stunde bei einer Badtemperatur von 170°C gerührt, wobei eine klare Lösung entsteht und dann ein Nebenprodukt ausfällt. Es wird heiß abgesaugt. Das Filtrat wird mit 5 ml Wasser versetzt und unter Rühren abgekühlt. Die ausgefallenen Kristalle werden abgesaugt, mit Wasser und Ethanol gewaschen und getrocknet. Man erhält 2,6 g (52,4 % der Theorie) farblose Kristalle vom Schmelzpunkt 227-229°C.
b)
   492,5 g (3 mol) 4-Aminophthalimidin (Rohware, enthält Katalysator) werden in 3 l Eisessig suspendiert und auf Rückfluß (115°C) erhitzt. Unter Rühren werden 497 ml (3 mol) Phenylacetaldehyddimethylacetal innerhalb von ca. 30 min zugetropft. Anschließend wird ca. 6 h am Rückfluß nachgerührt, es entsteht eine nahezu klare Lösung (bis auf den Katalysator). Das Reaktionsgemisch wird in der Hitze (ca. 80°C) filtriert und dann unter Rühren langsam auf Raumtemperatur abgekühlt. Die sich bildende Kristallmasse wird abgesaugt, mit ca. 500 ml Eisessig, dann mit ca. 100 ml Wasser gewaschen. Es wird 48 h bei ca. 70 bis 80°C im Vakuum getrocknet.
   Ausbeute: 383 g (65 % der Theorie)

### Beispiel 3

### 3-n-Hexylchinolin-5-carbonsäureamid

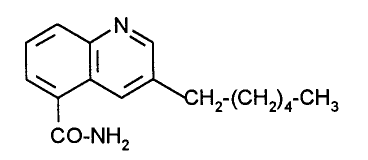

1,64 g (5 mmol) 4-Amino-3-hydroxyphthalimidin werden in 15 ml Diglyme mit 2 ml Octanal versetzt und 4 Stunden bei 160°C Badtemperatur gerührt. Es wird abgekühlt, auf Eiswasser gegeben, abgesaugt und mit Wasser und Petrolether gewaschen. Der Rückstand wird mit 10 ml warmen DMF verrührt und vom unlöslichen Rückstand filtriert. Das Filtrat wird durch Flash-Chromatographie mit Toluol/Aceton-Gemischen getrennt. Die Fraktionen, die die gewünschte Verbindung enthalten, werden eingeengt. Durch Kristallisation mit Acetonitril erhält man 130 mg (ca. 10 %) farblose Kristalle vom Schmelzpunkt 206 bis 208°C.

### Beispiel 4

### 3-Phenylchinolin-5-carbonitril

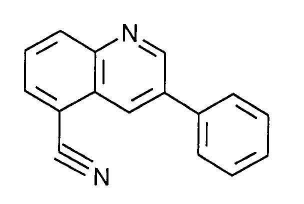

124 g (0,5 mol) der Verbindung aus Beispiel 2 (3-Phenylchinolin-5-carbonamid) werden in 500 ml DMF bei Raumtemperatur vorgelegt. Unter Rühren werden 90 ml Thionylchlorid unter Eiskühlung zugetropft, die Innentemperatur wird zwischen 20 bis 25°C gehalten. Es wird 2,5 h nachgerührt, dann 100 l Wasser zugetropft, und die Suspension in 1500 ml verdünnte Natronlauge (2,5 N) eingerührt. Die Temperatur wird durch Eiskühlung bei ca. 30°C gehalten. Das Kristallisat wird abgesaugt, mit Wasser gewaschen und im Vakuum getrocknet.
Ausbeute: 98 g (d.s. 85 % der Theorie)
IR (KBr): ϑ = 2220 cm⁻¹ (ϑ, CN).

### Anwendungsbeispiele

### (Herstellung des 3-Phenylchinolin-5-carbaldehyds)

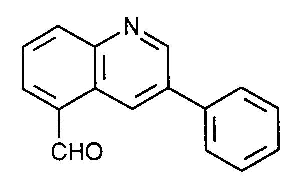

### Variante a:

200 g (0,75 mol) der Verbindung gemäß Beispiel 1c (Methylester) werden in 1500 ml tert.-Butanol gelöst. In diese Lösung werden 75 g Natriumboranat (2 mol) eingetragen. Darauf werden bei 70-75°C innerhalb von 3 Stunden 400 ml Methanol zugetropft (H₂-Entwicklung). Man läßt 30 Minuten unter Rückfluß nachrühren. Danach werden 50 ml Aceton zugetropft, und der Ansatz eine weitere Stunde unter Rückfluß gerührt. Zum Ansatz läßt man eine Mischung aus 450 ml Wasser und 50 ml konzentrierter Natronlauge laufen. Unter Normaldruck wird das Lösemittel bis zur Innentemperatur von 98-100°C abdestilliert (ca. 2000 ml). Nach Zugabe von 250 ml Wasser wird der Ansatz bei 50-60°C viermal mit je 300 ml Toluol extrahiert. Geringe Mengen teeriger Anteile sollen bei der wäßrigen Phase verbleiben. Die vereinigten toluolischen Lösungen werden unter Normaldruck auf ein Restvolumen von ca. 700 ml eingeengt und zu einer Suspension von 660 g Braunstein in 600 ml Toluol zugetropft. Der Ansatz wird darauf 3 h bei 45-50°C gerührt. Nach vollständiger Oxidation (HPLC-Kontrolle) wird der Braunstein abgesaugt und mit Toluol gewaschen. Das Toluol wird unter Vakuum weitgehend abdestilliert. Nach Zugabe von 650 ml Isopropanol wird das ausgefallene Produkt unter Rückfluß gelöst. Unter Normaldruck werden 150 ml Lösemittel abdestilliert. Der Ansatz wird auf 0°C abgekühlt und 2 h bei 0°C gerührt. Das Produkt wird abgesaugt mit 50 ml kaltem Isopropanol gewaschen und im Vakuum bei 30°C getrocknet.
Ausbeute: 113 g (64,6% d.Th.)

### Variante b:

23 g (0 mol) 3-Phenylchinolin-5-nithl (Substanz aus Beispiel 4) werden in 150 ml Ameisensäure mit 10 g Raney-Nickel versetzt. Es wird ca. 5 h bei ca. 80°C gerührt. Durch HPLC wird die langsame Bildung von 3-Phenylchinolin-5-aldehyd beobachtet. Daneben tritt als überreduziertes Produkt das 3-Phenyl-5-hydroxymethylchinolin auf. Zur Aufarbeitung wird in 300 ml H₂O eingerührt und gegebenenfalls mit 200 ml Methylenchlorid extrahiert. Die Methylenchlorid-Phasse wird anschließend 2 h mit 10 g Braunstein bei 40°C (Rückfluß) verrührt. Nach Filtration wird eingeengt und der Rückstand aus Isopropanol umkristallisiert.
Ausbeute: 13,5 g (= 58 % der Theorie).

## Patentansprüche

1. 3-substituierte Chinolin-5-carbonsäure-Derivate der allgemeinen Formel (I) 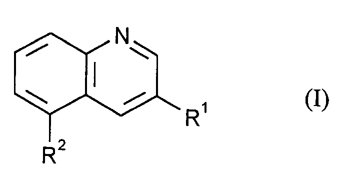 in welcher
R¹ für geradkettiges oder verzweigtes Alkyl mit bis zu 12 Kohlenstoffatomen steht, oder für einen Rest der Formel -X-R³ steht,
worin
X eine einfache Bindung, ein Sauerstoff- oder Schwefelatom oder eine Alkylidenkette mit bis zu 6 C-Atomen bedeutet,
und
R³ Cycloalkyl mit 3 bis 8 Kohlenstoffatomen oder Phenyl bedeutet, das gegebenenfalls bis zu 3-fach gleich oder verschieden durch Nitro, Halogen, Trifluormethyl oder durch geradkettiges oder verzweigtes Alkyl, Alkoxy oder Alkoxycarbonyl mit jeweils bis zu 8 Kohlenstoffatomen, Hydroxy oder Carboxy substituiert ist, und
R² für einen Rest aus der Gruppe -COOCH₃, -CN oder -CONH₂ steht.

2. Verbindungen der allgemeinen Formel (I) gemäß Anspruch 1, in welcher
R¹ für geradkettiges oder verzweigtes Alkyl mit bis zu 10 Kohlenstoffatomen steht, oder
für einen Rest der Formel -X-R³ steht,
worin
X eine einfache Bindung, ein Sauerstoff- oder Schwefelatom oder eine Alkylidenkette mit bis zu 4 C-Atomen bedeutet
und
R³ Cyclopropyl, Cyclopentyl, Cyclohexyl oder Phenyl bedeutet, das gegebenenfalls bis zu 2-fach gleich oder verschieden durch Nitro, Fluor, Chlor, Brom, Trifluormethyl oder durch geradkettiges oder verzweigtes Alkyl, Alkoxy oder Alkoxycarbonyl mit jeweils bis zu 6 Kohlenstoffatomen substituiert ist, und
R² für einen Rest aus der Gruppe -COOCH₃, -CN oder -CONH₂ steht.

3. Verbindungen der allgemeinen Formel (I) gemäß Anspruch 1, in welcher
R¹ für geradkettiges oder verzweigtes Alkyl mit bis zu 6 Kohlenstoffatomen steht oder für Cyclohexyl, Cyclohexylmethyl, Cyclopentyl steht oder für Phenyl steht, das gegebenenfalls bis zu 2-fach gleich oder verschieden durch Nitro, Fluor, Chlor, Trifluormethyl oder durch geradkettiges oder verzweigtes Alkyl, Alkoxy oder Alkoxycarbonyl mit jeweils bis zu 4 Kohlenstoffatomen substituiert ist, und
R² für einen Rest aus der Gruppe -COOCH₃, -CN oder -CONH₂ steht.

4. Verfahren zur Herstellung von Verbindungen der allgemeinen Formel (I) gemäß Anspruch 1, dadurch gekennzeichnet, daß man
[A] im Falle R² = COOCH₃ 3-substituierte Chinolin-5-carbonsäuren der allgemeinen Formel (II) 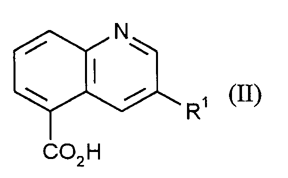 in welcher
R¹ die oben angegebene Bedeutung hat,
durch Umsetzung mit anorganischen oder organischen Säurechloriden gegebenenfalls nach Isolierung der entsprechenden Carbonsäurechloride diese mit Methanol verestert,
oder
[B] Carbonsäureamide der allgemeinen Formel (III) 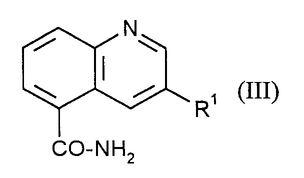 in welcher
R¹ die oben angegebene Bedeutung hat,
zunächst mit weitgehend wasserfreien Säuren in Methanol umsetzt und anschließend durch Zugabe von Basen die Ester ausfällt,
oder
[C] im Falle R² = -CO-NH₂ 4-Amino-3-hydroxyphthalimidin der Formel (IV) 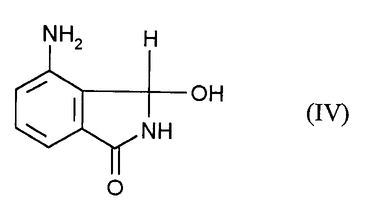 mit Aldehyden oder deren Acetal-Derivaten der allgemeinen Formel (Va) bzw. (Vb)
R¹-CH₂-CHO (Va) 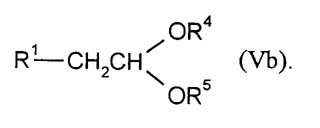
in welchen
R¹ die oben angegebene Bedeutung hat,
R⁴ und R⁵ gleich oder verschieden sind und für Cycloalkyl mit 3 bis 6 Kohlenstoffatomen, Aryl mit 6 bis 10 Kohlenstoffatomen, oder für geradkettiges oder verzweigtes Alkyl mit bis zu 6 Kohlenstoffatomen stehen, oder gemeinsam für eine Ethylen- oder Propylengruppe stehen,
in inerten Lösemitteln umsetzt und
[D] im Falle R² = -CN
die Amide der Formel III gegebenenfalls in Anwesenheit von inerten Lösemitteln durch Behandlung mit wasserentziehenden Mitteln in die Nitrile überführt.

5. Verwendung von Verbindungen der allgemeinen Formel (I) gemäß Anspruch 1 als Zwischenprodukte bei der Herstellung von Chinolin-5-carbaldehyden.
